# EUROPEAN PATENT SPECIFICATION

(11) **EP 1 525 878 B1**
(45) Date of publication and mention of the grant of the patent: **07.03.2007**
(21) Application number: 04024388.3
(22) Date of filing: 13.10.2004
(51) Int. Cl.: A61K 8/21, A61K 8/64, A61K 8/73, A61Q 11/00

(54) **Composition for caries prevention**
Zusammensetzungen zur Vorbeugung der Zahnkaries
Composition de prévention des caries

(30) Priority: 23.10.2003 JP 2003363014
(43) Date of publication of application: 27.04.2005
(73) Proprietor: GC Corporation, Itabashi-ku, Tokyo (JP)
(72) Inventor: Yamanaka, Katsuyuki, Tokyo (JP); Sato, Takuya, Tokyo (JP); Yoshi, Eiichi, Tokyo (JP); Watanabe, Yoshiko, Tokyo (JP)
(74) Representative: Müller-Boré & Partner Patentanwälte

(56) References cited:
- WO-A-98/40406
- US-A1- 2003 124 066

## Description

The present invention relates to a composition for oral cavity to prevent a caries, which has effects for suppressing tooth demineralization and promoting tooth remineralization.

Conventionally, as a component for the caries prevention, casein phosphopeptide - amorphous calcium phosphate complex (hereinafter, it is referred to as CPP - ACP) has been known. The CPP - ACP is a material comprising a complex of peptide derived from milk protein (casein) and an inorganic material (an amorphous calcium phosphate), and it has been said that the CPP - ACP act on demineralization and remineralization of the tooth since it contains calcium and phosphorus, which are inorganic components of the tooth, as the constituent elements (for example, refer to non-patent literature 1, Reynolds EC et al. "Anticariogenicity of calcium phosphate complexes of tryptic casein phosphopeptides in the rat" J Dent Res. 1995 Jun : 74(6) : 1272-9, and non-patent literature 2, Reynolds EC "Remineralization of enamel subsurface lesions by casein phosphopeptide - stabilized calcium phosphate solutions" J Dent Res. 1997 Sep : 76 (9) : 1587-95.)

As for the caries prevention with the CPP - ACP, two processes comprising an acceleration of remineralization action and an inhibition of demineralization action are considered. The acceleration of remineralization action can be explained by the results, where the concentrations of calcium and phosphorus can be continuously increased and kept in the inside of a demineralized lesion under the enamel subsurface, by continuously supplying the CPP - ACP. Furthermore, the inhibition of demineralization action is based on the following logic, where the solubility of calcium phosphate in the enamel subsurface is remarkably decreased since the ion concentration in saliva on the enamel subsurface or the inside of a dental plaque is kept in the supersaturated state with the ions of calcium and phosphorus came from the CPP - ACP, and then, the tooth becomes to be hardly melted.

Moreover, it has been known that fluorides have many effects for promoting the remineralization, improving an acid resistance of tooth structure, and suppressing an acid production with exhibiting antimicrobial property to plaque bacteria, and thus a casein phosphopeptide - amorphous calcium fluoride phosphate complex (hereinafter it is referred to as CPP - ACFP), which contains fluorides, has also effect for the caries prevention.

The foods, such as a chewing gum and a candy, and the compositions for oral care, such as a dentifrice and a mouth washing agent, which assist the caries prevention by containing the CPP - ACP and CPP - ACFP having the above action, have been disclosed (for example, refer to patent literature 1, published Japanese translation of PCT international publication for patent application 2002- 500626, and patent literature 2, published Japanese translation of PCT international publication for patent application 2002- 540129).

However, in order to demonstrate the preventing effect of the caries with the CPP - ACP in the oral cavity, it is necessary to keep the component of the CPP - ACP for a long time. For example, in the case of the chewing gum blended with the CPP - ACP, it is necessary to chew simultaneously two grains of it, i.e., 3 grams, for 20 minutes and four times per day during two weeks as a standard (refer to non-patent literature 3, Shen P et al. "Remineralization of enamel subsurface lesions by sugar-free chewing gum containing casein phophopeptide - amorphous calcium phosphate." J Dent Res 2001 Dec : 80(12) : 2066-70).

It is difficult to apply such action many times per day as mentioned above when considering the general lifestyle. So, in order to demonstrate the prevention effect of the caries with the CPP - ACP even when applying 1 time per day, it has been examined to apply the composition for caring the oral cavity like a paste or a cream, which contains the CPP - ACP, on the tooth.

However, as for the conventional composition for caring the oral cavity like the paste or the cream, which contains the CPP - ACP, there is a problem that the state of the composition becomes uneven since the composition is gelled or lumps are generated at the time of preserving for a long time.

Then, the present invention has the object to provide the composition for the caries prevention, which has the effects for efficiently suppressing the demineralization and promoting the remineralization, and an excellent stability in keeping for a long time.

The present inventors made wholeheartedly investigations in order to solve the above problems, and as the result, they found out the method to obtain the excellent composition for the caries prevention. That is, in the composition comprising (a) the casein phosphopeptide - amorphous calcium phosphate complex (CPP - ACP) and / or the casein phosphopeptide - amorphous calcium fluoride phosphate complex (CPP - ACFP), (b) a sodium carboxymethylcellulose, (c) a viscosity regulator, and (d) water, when the sodium carboxymethylcellulose having a specific etherification degree was used, the composition for the caries prevention, which has the effects for efficiently suppressing the demineralization and promoting the remineralization, and the excellent stability in keeping for a long time, was obtained. Then, the present invention was completed.

In a first aspect of the present invention, composition for the oral cavity to prevent the caries comprises (a) 1 to 30 % by weight of the casein phosphopeptide - amorphous calcium phosphate complex (CPP - ACP) and / or the casein phosphopeptide - amorphous calcium fluoride phosphate complex (CPP - ACFP), (b) 0.01 to 10 % by weight of the sodium carboxymethylcellulose, having the etherification degree of 0.7 to 1.0, (c) 0.5 to 40 % by weight of the viscosity regulator, and (d) a residual amount of water. This composition is used by coating on the tooth. In a second aspect of the present invention, a composition for oral cavity to prevent a caries comprises (a) 1 to 30 % by weight of the casein phosphopeptide-amorphous calcium phosphate complex (CCP-ACP) and/or the casein phosphopeptide-amorphous calcium fluoride phosphate complex (CCP-ACFP), (b) 0.01 to 10 % by weight of the sodium carboxymethylcellulose having the etherification degree of 0.7 to 1.0, (c) 0.5 to 40 % by weight of the viscosity regulator, (e) 0.01 to 5.0 % by weight of the fluoride compound, and (d) a residual amount of water, and being used by applying on a tooth. In a third aspect of the present invention, the fluoride compound is at least one selected from the group consisting of sodium fluoride, stannous fluoride, sodium monofluoro phosphate in the composition for oral cavity to prevent a caries according to the second aspect of the present invention.

Hereinafter, the composition for the caries prevention of the present invention will be explained. (a) The casein phosphopeptide - amorphous calcium phosphate complex (CPP - ACP) and / or the casein phosphopeptide - amorphous calcium fluoride phosphate complex (CPP - ACFP) are not especially limited, if it is approved as foods or in dentistry. For example, the material prepared by the method of mixing calcium, an inorganic phosphoric acid, and fluoride if necessary with phosphopeptide, filtrating the mixed material, and drying, is preferably used in respect of the composition and crystallinity (refer to published Japanese translation of PTC international publication for patent application 2002-500626).

At this time, the blending amount of the casein phosphopeptide - amorphous calcium phosphate complex (CPP-ACP) and / or the casein phosphopeptide - amorphous calcium fluoride phosphate complex (CPP - ACFP) (a) is 1 to 30 % by weight of the whole composition, preferably 1 to 10 % by weight especially. If said amount is less than 1 % by weight, the supplying of calcium and phosphorus is not sufficient, so that the prevention effect of the caries can not be obtained. If said amount exceeds 30 % by weight, the viscosity of the composition becomes too high.

The etherification degree of the sodium carboxymethylcellulose (b), which is blended to obtain stability in keeping for a long time, is 0.7 to 1.0, preferably 0.85 to 0.95 especially. Because, if the etherification degree is less than 0.7, the blending effect can not be obtained, and if said amount exceeds 1.0, the sodium carboxymethylcellulose is easily influenced by calcium existing in the CPP - ACP and / or the CPP - ACFP, so that the preservation property becomes to be insufficient, since an insoluble precipitate is generated or said sodium carboxymethylcellulose is gelled.

The blending amount of the sodium carboxymethylcellulose having the etherification degree of 0.7 to 1.0 is 0.01 to 10 % by weight of the whole composition, preferably 1 to 5 % by weight especially. If said amount is less than 0.01 % by weight, the viscosity of the composition is low and it easily flows with saliva at the time of the applying on the tooth. Therefore, the prevention effect of the caries can not be obtained. If said amount exceeds 10 % by weight, the viscosity of the composition is high and it can not easily spread at the time of the applying on the tooth, and the sense of use is also bad.

In the composition for the caries prevention of the present invention, it is necessary to use (c) the viscosity regulator, in order to use the composition by applying on the tooth and to obtain the prevention effect of the caries by (a) the casein phosphopeptide - amorphous calcium phosphate complex (CPP-ACP) and / or the casein phosphopeptide - amorphous calcium fluoride phosphate complex (CPP - ACFP) being stayed in the oral cavity. As for (c) the viscosity regulator, for example, a natural material, such as gum guaiac, carob bean gum, Tara gum, tamarind seed gum, gum arabic, tragacanth gum, karaya gum, carrageenan, xanthan gum, gellan gum, curdlan, chitin, chitosan or chitosamine, an organic synthetic material, such as glycerin, propylene glycol, sodium alginate, propylene glycol ester alginate, sodium starch glycolate, sodium starch phosphate ester, sodium polyacrylate or polyvinyl pyrrolidone, or an inorganic synthetic material, such as calcium carbonate, calcium silicate, a silica fine powder, amorphous hydrous silica or hydrophobic silica, can be used.

When the using method of directly applying on the tooth is considered, the viscosity of the composition is suitably 1,000 to 500,000 cP at 25 degree C, preferably 20,000 to 200,000 cP. Therefore, the blending amount of (c) the viscosity regulator is 0.5 to 40 % by weight of the whole composition, preferably 1 to 10 % by weight especially.

The present invention is of the composition for the oral cavity to prevent the caries, further comprising (e) 0.01 to 5.0 % by weight of a fluoride complex, in order to obtain more caries prevention. If said amount is less than 0.05 % by weight, the acceleration of remineralization is low and if said amount exceeds 5.0 % by weight, the safety to human body becomes low. Preferable amount is 0.05 to 2.0 % by weight. The fluoride complex is preferably used at least one selected from the group consisting of sodium fluoride, stannous fluoride, sodium monofluoro phosphate.

Since the composition for the caries prevention of the present invention is directly coated on the tooth, pH is adjusted in the range of 5.5 to 9.5 preferably. Although the method for keeping said range is not limited especially, for example, an aqueous solution of phosphate, which is one of the constitution components of the CPP - ACP and the CPP - ACFP, or phosphate salt is added to adjust the pH preferably.

In addition, in the composition for the caries prevention of the present invention, a sweetener, a colorant, a preservative, an antifungal agent, or a flavor etc., which are used to the general composition for the caries prevention, can be suitably blended.

Hereinafter, the present invention will be explained concretely with the examples and comparison examples.

The blends of the compositions used in the examples and the comparison examples were collectively shown in Table 1 and 2, and the pH and the viscosity at 25 degree C of these compositions were collectively shown in Table 3 and 4.

### [Evaluation of Keeping Stability]

The compositions for the oral cavity to prevent the caries, which were blended with the composition shown in Table 1 and 2, were kept under the conditions at 60 degree C for 1 week, and then, said compositions were observed. These results were collectively shown in Table 3 and 4.

### [Evaluation of Remineralization Effect]

The evaluation was carried out by using a bovine tooth in vitro, according to the method described in Reynolds EC "Remineralization of enamel subsurface lesions by casein phosphopeptide-stabilized calcium phosphate solutions." J. Dent. Res. 1997 Sep; 76(9): 1587-95.
(1) The bovine tooth was embedded with a dental resin to make an enamel testing surface having the length of 7 mm and the width of 1 mm. Said enamel testing surface was made into an enamel block.
(2) The enamel block was dipped in 40 ml of the demineralization liquid, which was blended with following materials, under an atmosphere at 37 degree C for 4 days, to make an artificial caries.
   Blending of the demineralization liquid (pH 4.8) lactic acid : 0.1 mol / L
   calcium chloride : 1.5m.mol/ L
   potassium dihydrogen phosphate : 0.9m.mol / L
   sodium carboxymethylcellulose (cellogen HE-1500F, made by Daiichi Kogyo Seiyaku Co., Ltd.) : 1% by weight
(3) An enamel varnish was coated on about a half of the enamel block surface, where the artificial caries was made, for not generating the remineralization, and said enamel block was dipped in a slurry liquid, which was obtained by diluting the compositions of Examples 1~20 and Comparison examples 1~4 with distilled water 5 times, for 10 days, to carry out the remineralization treatment. At the Examples18, 19 and 20, a phosphatase (Phosphatase, acid derived from wheat, made by SIGMA Chem. Co., Ltd.) is added 0.025 units/mL to said slurry liquid.
(4) A thin sliced piece having the thickness of about 100pm was made from the enamel block.
(5) A X-ray picture of the thin sliced piece of (4) was photographed, and mineral qualities of demineralized and remineralized parts were calculated with the distances from the surface and the luminances at said parts, by using an image analyzer. Then, a remineralization ratio was obtained. If the value of said ratio was larger, such part was more remineralized. These results were collectively shown in Table 3 and 4.

### [Evaluation of Staying Ability in Oral Cavity]

The composition for the caries prevention blended with the composition shown in Table 1 and 2 is coated on the whole surface of the tooth by a tooth brush. After one hour, the calcium ion in the saliva was measured to evaluate the staying ability. These results were shown in Table 3 and 4.

**[Table 1]**

| | Component | Example 1 | Example 2 | Example 3 | Example 4 | Example 5 | Example 6 | Example 7 | Example 8 | Example 9 | Example 10 |
|---|---|---|---|---|---|---|---|---|---|---|---|
| (a) | CPP - ACP | 10.00 | | 10.00 | 5.00 | 20.00 | 10.00 | 5.00 | 10.00 | 10.00 | 10.00 |
| | CPP - ACFP | | 10.00 | | 5.00 | | | | | | |
| (b) | Cellogen F - SA (Etherification Degree is 0.70 to 0.80) | | | 3.50 | | | 1.25 | | 0.50 | | 1.00 |
| | Cellogen F - AG (Etherification Degree is 0.85 to 0.95) | 3.00 | 3.00 | | 3.00 | 2.00 | 1.25 | 2.00 | | 2.50 | 1.00 |
| | Cellogen F - 1220B (Etherification Degree is 1.15 to 1.35) | | | | | | | | | | |
| | Cellogen F - 90F (Etherification Degree is 1.20 to 1.30) | | | | | | | | | | |
| (c) | Guam Guaiac | | | | | | | 0.50 | | | |
| | Xanthan Gum | | | | | | | | 1.00 | | |
| | Tamarind Gum | | | | | | | | | 0.50 | |
| | Rapport Gum | | | | | | | | | | 0.30 |
| | Glycerin | 20.00 | 20.00 | 15.00 | 20.00 | 20.00 | 10.00 | 30.00 | 20.00 | 15.00 | 20.00 |
| | Propylene Glycol | 2.00 | 2.00 | 10.00 | 2.00 | 3.00 | 5.00 | 0.50 | 2.00 | 1.00 | 2.00 |
| | Polyvinyl Pyrrolidone | | | | | | 1.00 | | | | |
| | Aerosil A200 | 2.00 | 2.00 | 2.00 | 2.00 | 1.50 | 2.50 | 2.00 | 2.00 | 2.00 | 2.00 |
| | Titanium Dioxide | 1.50 | 1.50 | 1.50 | 2.00 | 2.50 | 2.00 | 2.50 | 2.00 | 2.00 | 2.00 |
| addutude | Xylitol | 1.00 | 1.00 | 1.00 | 1.00 | 1.00 | 1.00 | 1.00 | 1.00 | 1.00 | 1.00 |
| | Sorbitol | 0.10 | 0.10 | 0.10 | 0.10 | 0.10 | 0.10 | 0.10 | 0.10 | 0.10 | 0.10 |
| | p-Hydroxy Benzoic Ester | 0.10 | 0.10 | 0.10 | 0.10 | 0.05 | 0.10 | 0.10 | 0.10 | 0.10 | 0.10 |
| | Spice | 0.20 | 0.20 | 0.20 | 0.20 | 0.10 | 0.20 | 0.20 | 0.20 | 0.20 | 0.20 |
| | Phosphate | 0.30 | 0.30 | 0.30 | 0.30 | 0.40 | 0.15 | 0.30 | 0.30 | 0.30 | 0.30 |
| (d) | Water | 59.80 | 59.80 | 56.30 | 59.30 | 49.35 | 65.45 | 55.80 | 60.80 | 65.30 | 60.00 |

| | | | | | | | | | | | |
|---|---|---|---|---|---|---|---|---|---|---|---|
| (Note) Cellogen F-SA, Cellogen F-AG, Cellogen F-1220B and Cellogen F-90F are sodium carboxymethylcellulose produced by Daiichi Kogyo Seiyaku Co., Ltd. Aerosil A200 is silica fine powder produced by Nippon Aerosil Co., Ltd. | | | | | | | | | | | |

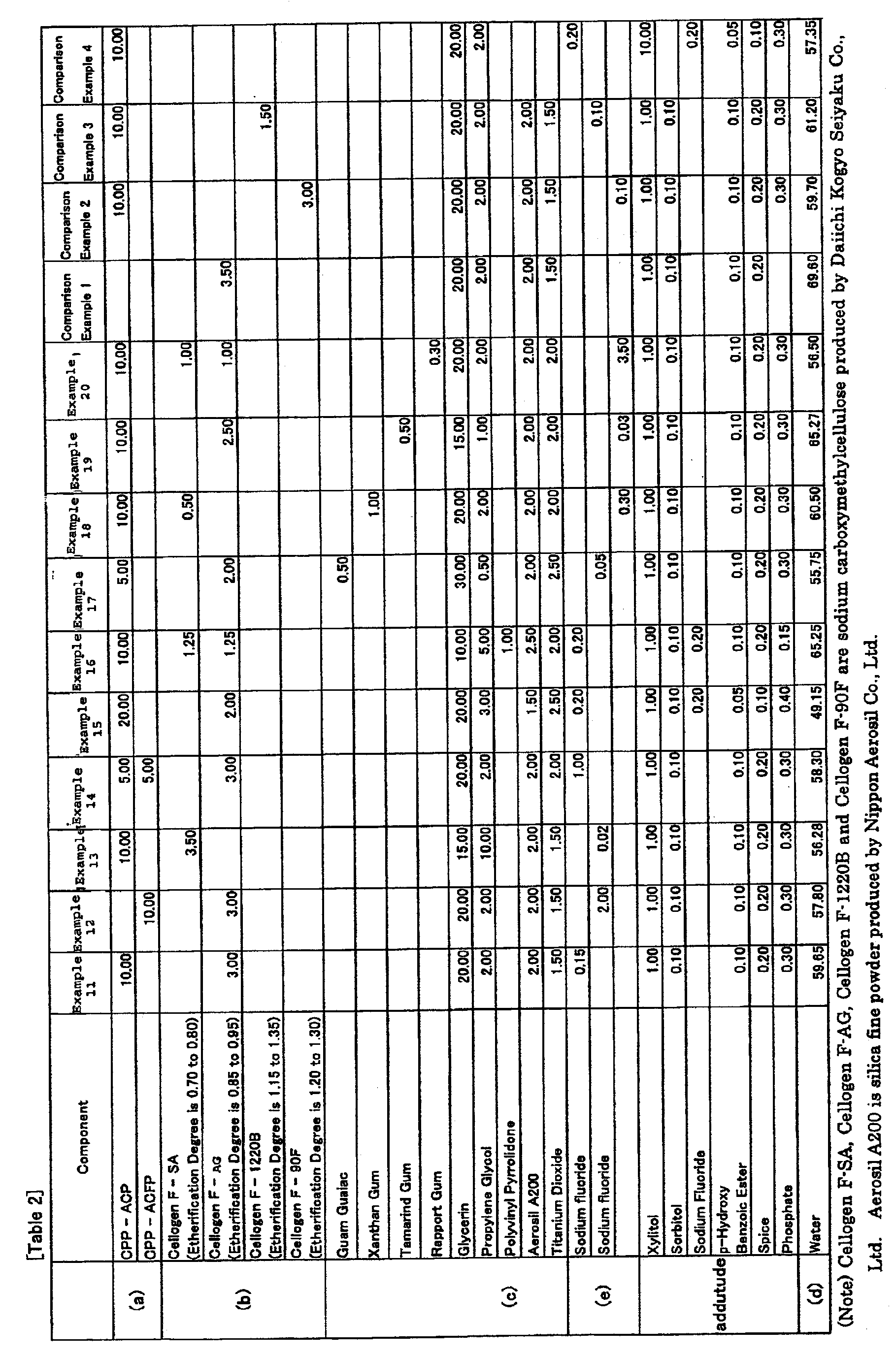

**[Table 3]**

| | Example 1 | Example 2 | Example 3 | Example 4 | Example 5 | Example 6 | Example 7 | Example 8 | Example 9 | Example 10 |
|---|---|---|---|---|---|---|---|---|---|---|
| pH | 7.8 | 7.8 | 7.8 | 7.8 | 7.5 | 7.8 | 7.8 | 7.8 | 7.8 | 7.8 |
| Viscosity [cp / 25 degree C] | 70,000 | 5,000 | 58,000 | 68,000 | 71,000 | 75,000 | 60,000 | 52,000 | 85,000 | 66,000 |
| Keeping Stability [Keeping at 60 degree C for 1 week] | Good | Good | Good | Good | Good | Good | Good | Good | Good | Good |
| Remineralization Ratio [%] | 35.2 | 49.8 | 30.5 | 39.3 | 54.3 | 43.3 | 29.8 | 33.9 | 31.8 | 39.4 |
| Staying Ability in Oral Cavity [µg/mL] | 92.5 | 90.1 | 89.7 | 91.2 | 115.4 | 98.3 | 78.2 | 84.5 | 93.3 | 96.7 |

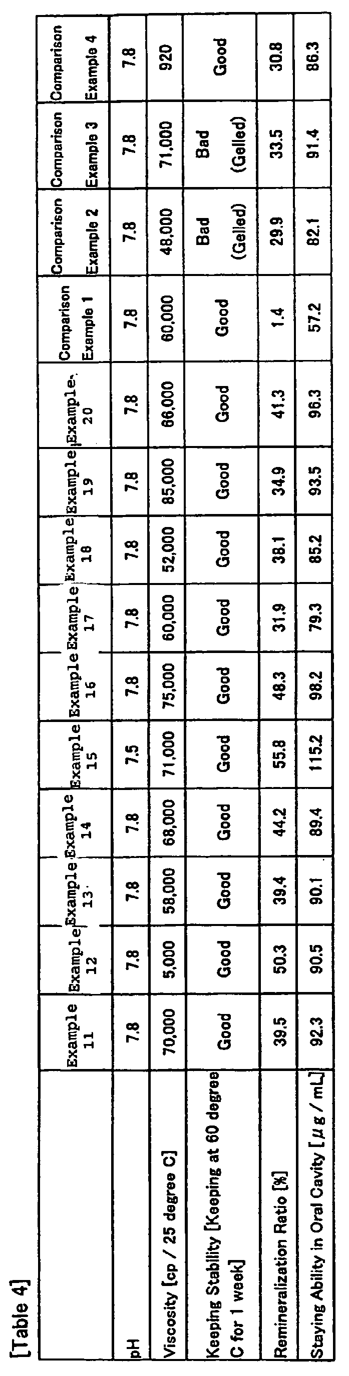

Clearly from Table 3, it was identified that the composition for the caries prevention being excellent in the caries prevention and the stability in keeping for a long time, could be obtained, by selecting the specific sodium carboxymethylcellulose having the specific etherification degree and blending said sodium carboxymethylcellulose with the composition for the caries prevention containing the CPP - ACP and / or CPP-ACFP.

The composition for the caries prevention of the present invention contains the CPP - ACP and / or CPP-ACFP and is blended with sodium carboxymethylcellulose having the etherification degree of 0.7 to 1.0, so that it has promoting effect of the remineralization and excellent stability in keeping for a long time. Therefore, said composition has the great value for contributing to dental medicine.

Clearly from Table 4, the compositions which are further containing the fluoride complex, are excellent in the caries prevention as compared with the compositions which have no fluoride complex as in Examples 1 to 10.

## Claims

1. A composition for oral cavity to prevent a caries, comprising (a) 1 to 30 % by weight of a casein phosphopeptide - amorphous calcium phosphate complex (CPP - ACP) and / or a casein phosphopeptide - amorphous calcium fluoride phosphate complex (CPP - ACFP), (b) 0.01 to 10 % by weight of a sodium carboxymethylcellulose having an etherification degree of 0.7 to 1.0, (c) 0.5 to 40 % by weight of a viscosity regulator, and (d) a residual amount of water, and being used by applying on a tooth.

2. A composition for oral cavity to prevent a caries, comprising (a) 1 to 30 % by weight of a casein phosphopeptide-amorphous calcium phosphate complex (CPP -ACP) and/or a casein phosphopeptide-amorphous calcium fluoride phosphate complex (CPP-ACFP), (b) 0.01 to 10 % by weight of a sodium carboxymethylcellulose having an etherification degree of 0.7 to 1.0, (c) 0.5 to 40 % by weight of a viscosity regulator, (e) 0.01 to 5.0 % by weight of a fluoride compound, and (d) a residual amount of water, and being used by applying on a tooth.

3. A composition for oral cavity to prevent a caries as claimed in Claim 2, wherein the fluoride compound is at least one selected from the group consisting of sodium fluoride, stannous fluoride, sodium monofluoro phosphate.

## Patentansprüche

1. Zusammensetzung für die Mundhöhle, um einer Karies vorzubeugen, umfassend (a) 1 bis 30 Gew.-% eines Caseinphosphopeptid-amorphes Calciumphosphat-Komplexes (CPP - ACP) und/oder eines Caseinphosphopeptid-amorphes Calciumfluoridphosphat-Komplexes (CPP - ACFP), (b) 0,01 bis 10 Gew.-% einer Natriumcarboxymethylcellulose mit einem Veretherungsgrad von 0,7 bis 1,0, (c) 0,5 bis 40 Gew.-% eines Viskositätsregelmittels und (d) eine restliche Menge an Wasser, und welche durch Aufbringen auf einen Zahn verwendet wird.

2. Zusammensetzung für die Mundhöhle, um einer Karies vorzubeugen, umfassend (a) 1 bis 30 Gew.-% eines Caseinphosphopeptid-amorphes Calciumphosphat-Komplexes (CPP - ACP) und/oder eines Caseinphosphopeptid-amorphes Calciumfluoridphosphat-Komplexes (CPP - ACFP), (b) 0,01 bis 10 Gew.-% einer Natriumcarboxymethylcellulose mit einem Veretherungsgrad von 0,7 bis 1,0, (c) 0,5 bis 40 Gew.-% eines Viskositätsregelmittels, (e) 0,01 bis 5,0 Gew.-% einer Fluoridverbindung und (d) eine restliche Menge an Wasser, und welche durch Aufbringen auf einen Zahn verwendet wird.

3. Zusammensetzung für die Mundhöhle, um einer Karies vorzubeugen, nach Anspruch 2, wobei die Fluoridverbindung mindestens eine, ausgewählt aus der Gruppe, bestehend aus Natriumfluorid, Zinnfluorid, Natriummonofluorphosphat, ist.

## Revendications

1. Composition pour cavité buccale destinée à empêcher une carie, comprenant (a) 1 à 30% en poids d'un complexe de phosphopeptide de caséine et de phosphate de calcium amorphe (CCP-ACP) et/ou d'un complexe de phosphopeptide de caséine et de phosphate fluorure de calcium amorphe (CCP-ACFP), (b) 0,01 à 10% en poids d'une carboxyméthycellulose de sodium ayant un degré d'éthérification de 0,7 à 1,0, (c) 0,5 à 40% en poids d'un régulateur de viscosité et (d) une quantité résiduelle d'eau, et utilisée par application sur une dent.

2. Composition pour cavité buccale destinée à empêcher une carie, comprenant (a) 1 à 30% en poids d'un complexe de phosphopeptide de caséine et de phosphate de calcium amorphe (CCP-ACP) et/ou d'un complexe de phosphopeptide de caséine et de phosphate fluorure de calcium amorphe (CCP-ACFP), (b) 0,01 à 10% en poids d'une carboxyméthycellulose de sodium ayant un degré d'éthérification de 0,7 à 1,0, (c) 0,5 à 40% en poids d'un régulateur de viscosité, (e) 0,01 à 5,0% en poids d'un composé de fluorure et (d) une quantité résiduelle d'eau, et utilisée par application sur une dent.

3. Composition pour cavité buccale destinée à empêcher une carie selon la revendication 2, dans laquelle le composé de fluorure est au moins un composé choisi dans le groupe constitué du fluorure de sodium, du fluorure stanneux et du monofluorophosphate de sodium.
